# EUROPEAN PATENT APPLICATION

(11) **EP 0 574 989 A1**
(43) Date of publication of application: **22.12.1993**
(21) Application number: 93201651.2
(22) Date of filing: 10.06.1993
(51) Int. Cl.: C01B 21/14, C01B 21/40

(54) **Process for the preparation and processing of a hydroxylammonium salt solution**

(30) Priority: 16.06.1992 NL 9201064
(71) Applicant: DSM N.V., NL-6411 TE Heerlen (NL)
(72) Inventor: Rouhof, Hendrikus Johannes Hermanus, NL-6132 SG Sittard (NL); van Dortmont, Godefridus Maria, NL-6127 CR Born (NL); Boesten, Michael Wilhelmus Maria, NL-6191 VS Beek (L) (NL)

(57) **Abstract**

The invention relates to a process for the preparation and processing of a hydroxylammonium salt solution, wherein an aqueous acid reaction medium is kept in circulation between a hydroxylammonium salt synthesis zone (A) and an oxime synthesis zone (B) and wherein nitrate ions or nitrogen oxides to be converted into nitrate are continuously supplied (12'), by way of nitrogen source for the formation of the hydroxylammonium salt, to the liquid being kept in circulation (via 16'), which nitrate ions are catalytically reduced with hydrogen (19') to hydroxylamine, and wherein ammonium ions, formed as a by-product in the reduction of the nitrate ions, are removed by contacting the circulating liquid (18') in an absorber system (C) with a gas flow (13') consisting of nitrogen oxides generated in catalytic combustion of ammonia, 0.01 to 5 wt.% of the nitrogen oxides formed in the combustion being used to reduce the ammonium ions to nitrogen, and the rest of the nitrogen oxides being used (via 15') for the preparation of nitric acid.

## Description

The invention relates to a process for the preparation and processing of a hydroxylammonium salt solution, wherein an aqueous acid reaction medium is kept in circulation between a hydroxylammonium salt synthesis zone and an oxime synthesis zone and wherein nitrate ions or nitrogen oxides to be converted into nitrate ions are continuously supplied, by way of nitrogen source for the formation of the hydroxylammonium salt, to the liquid being kept in circulation, which nitrate ions are catalytically reduced with hydrogen to hydroxylamine, and wherein ammonium ions, formed as a by-product in the reduction of the nitrate ions, are removed by conversion with nitrogen oxides.

In a process according to the invention, cyclohexanone oxime is produced starting from hydroxylammonium salt and cyclohexanone, which cyclohexanone oxime can be used as a starting material for the preparation of ε-caprolactam. Such a process is described in GB-A-1287303. That process is characterized by a circulation of a buffered solution between a zone wherein hydroxylammonium salt is formed and a zone wherein cyclohexanone oxime is formed. The chemical reactions that take place in the different zones are represented by reaction equations 1-3.
1) Preparation of the hydroxylammonium salt:

   2 H₃PO₄ + NO₃⁻ + 3 H₂ → NH₃OH⁺ + 2 H₂PO₄⁻ + 2 H₂O (1)
2) Preparation of the oxime: 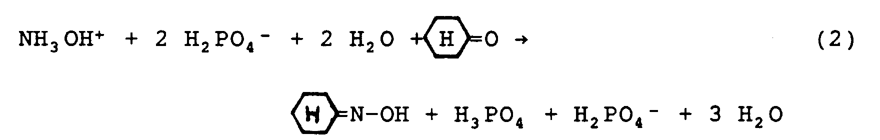
3) Supply of HNO₃ to make up the depletion of the source of nitrate ions after removal of the oxime formed:

   H₂PO₄⁻ + HNO₃ → H₃PO₄ + NO₃⁻ (3)

The depletion of the nitrate ion source is made up by absorption of gases containing nitrogen oxides, resulting in formation of nitric acid, into the reaction mixture that is kept in circulation. Directly supplying of nitric acid to the reaction mixture is also possible. It is further possible to combine the absorption of nitrogen oxides and the supplying of nitric acid. When the nitric acid or the nitrogen oxides to be converted into nitrate have been added, the reaction mixture, after the removal of water, has the same theoretical composition as the initial solution started from for the preparation of the hydroxylammonium salt. It is also known, however, that in the step involving the preparation of the hydroxylammonium salt a quantity of ammonium ions is formed owing to the hydrogenation not being fully selective. In order to avoid an accumulation of ammonium ions in a continuous circulation process, the ammonium ions formed are converted to nitrogen by means of gases containing nitrogen oxides in accordance with the following reaction:

2 NH₄⁺ + NO + NO₂ → 2 N₂ + 3 H₂O + 2 H⁺ (4)

This process is known as the HPO-process (Hydroxylamine Phosphate Oxime).

The nitrogen oxides (nitrogen monoxide + nitrogen dioxide) are obtained in accordance with the state of the art by on-purpose ammonia combustion, whereby ammonia and air are converted into nitrogen oxides and water. Part of the nitrogen monoxide formed is oxidized to nitrogen dioxide by means of secondary air. A drawback of this process is that the efficiency of the ammonia combustion forming part of a process with a capacity that is comparable to that of a normal HPO process is in general only 91% (on a per mol basis).

The object of the invention is to improve the efficiency of the ammonia combustion.

This object is achieved in that the circulating liquid containing ammonia ions is contacted with a gas flow containing nitrogen oxides generated in catalytic combustion of ammonia, 0.01 to 5 wt.% of the nitrogen oxides formed in the combustion being used to convert ammonium ions into nitrogen, and in that the rest of the nitrogen oxide containing gas flow is used for the preparation of nitric acid.

Preferably, 0.5-3 wt.% of the nitrogen oxides is used to convert ammonium ions into nitrogen.

The ratio between the quantity of nitrogen oxides that are used to convert ammonium ions into nitrogen (by reaction 4) and the quantity that is used for the preparation of the hydroxylammonium salt solution (by nitric acid formation and reaction 1) is in general between 5:95 and 25:75.

The process according to the invention can be applied for the preparation of oximes with 2-20 carbon atoms. Preferably the hydroxyl ammonium salt preparation is used for the preparation of cycloalkanone oximes with 6-12 carbon atoms. The invention is very suitable for a process in which cyclohexanone oxime is prepared because cyclohexanone oxime is an intermediate in a process to prepare ε-caprolactam.

Application of the invention makes it possible to use a single ammonia combustion plant for the preparation of nitrogen oxide for the preparation of a hydroxylammonium salt solution in a commercial HPO process, and at the same time for the preparation of nitric acid in a commercial nitric acid production process. By a commercial nitric acid production process is meant a process with a capacity of 200 to 750 kt HNO₃/year (on dry basis). A commercial HPO process in general has a nitric acid consumption of 50 to 110 kt/HNO₃/year (on dry basis). This nitric acid consumption corresponds to the total of nitric acid and nitrogen oxides (to be converted into nitric acid) that is consumed in an HPO process.

The advantage achieved in this way is that the ammonia combustion can be carried out on such a scale that the efficiency of the ammonia combustion will be considerably higher than is the case when ammonia combustion is carried out only for the HPO process.

In GB-A-1287303 it is described how and with what process steps the nitrogen oxide containing flow and the nitric acid are produced in an HPO process. From that description it already appears that much process equipment is required for such a process. In addition to the steps described therein, purification steps are required in order to meet today's environmental standards, which means that in practice the number of process steps will be even greater. The advantage that is achieved by applying the present invention is that the number of process steps in the HPO process can be reduced. The number of times that the ammonia combustion facility has to be put on and taken off stream will be lower than in the situation where there is an ammonia combustion facility for the HPO process and a separate ammonia combustion facility for a nitric acid production process. The environmental impact from putting on and taking off stream the ammonia combustion facility will therefore be reduced by application of the process according to the invention.

A first embodiment of the invention is a process wherein part of the nitrogen oxide containing flow from a nitric acid production process is used for the destruction of ammonium ions in a consecutive step, the nitrogen oxides that are not converted in said ammonium ion destruction step being used on the spot for the preparation of nitric acid to be used in the hydroxylammonium salt preparation step. This means that next to a simply realizable ammonium ion destruction step a number of absorption columns are needed for this nitric acid production.

A second - preferred - embodiment of the invention is a process wherein a nitrogen oxide containing flow from a (nearby situated) nitric acid production process is used for the destruction of ammonium ions and wherein the non-converted nitrogen oxides are recycled to the nitric acid production process. The nitrate ions for the preparation of the hydroxylammonium salt are also obtained from the nitric acid production process, in the form of nitric acid. Application of this process means that no absorption columns are used for the nitric acid preparation in the HPO-process. If desired, the circulating liquid containing ammonium ions could be supplied through a line to the nitric acid preparation process section. The ammonium ion destruction step can then be effected by using a nitrogen oxide containing flow from the nitric acid preparation process.

The process according to the prior art and a preferred embodiment of the process according to the invention will be illustrated by means of figures in the following, without being restricted thereto.

Figure 1 shows a block diagram of a process according to the prior art.

Figure 2 shows a block diagram of a process according to the invention.

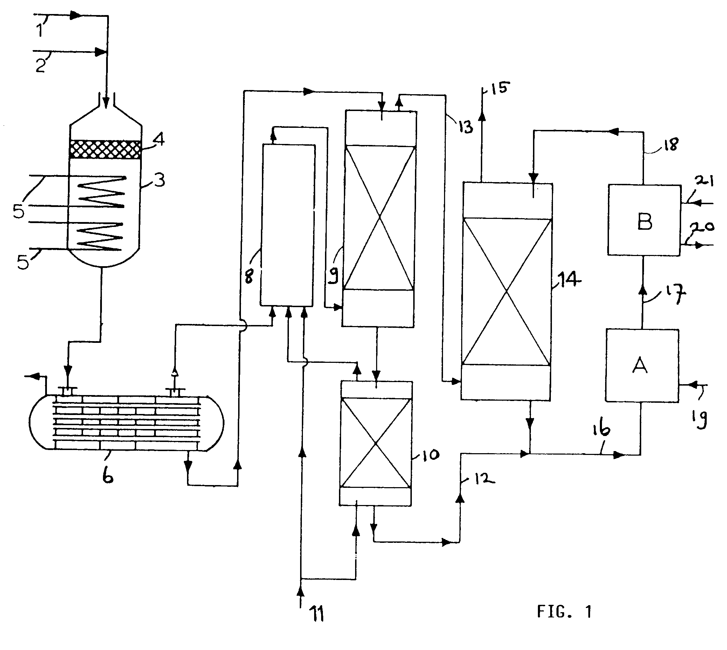

Figure 1 and the description thereof are derived from GB-A-1287303. In Figure 1 the nitric acid and nitrogen oxide production is represented by an ammonia combustion facility 3 equipped with a platinum gauze 4 and steam pipes 5, a hot gas condenser 6, oxidation column 8, absorption system 9, which in general comprises several columns, and stripping column 10. Nitric acid and a nitrogen oxide containing gas flow leave this section via line 12 and line 13, respectively, while oxygen, ammonia and secondary air are supplied via lines 1, 2 and 11, respectively.

The hydroxylammonium salt synthesis zone is represented by zone A, to which hydrogen is supplied via line 19. An oxime synthesis zone B where the oximation reaction and the oxime extraction take place has a supply line 21 for the ketone (cyclohexanone) and an outlet 20 for the oxime. Line 18, absorption system 14, which will in general comprise several absorption columns and oxidation reactors and lines 16 and 17 complete the recycling system, wherein the reaction medium is recycled between zone A and zone B.

In absorption system 14 the reaction medium is contacted with nitrogen oxides (via line 13). This results in, among other things, destruction of the ammonium ions and formation of nitric acid by absorption of nitrogen dioxide. The non-converted nitrogen oxides are discharged via line 15.

In this embodiment the combustion of ammonia to nitrogen oxides is carried out at atmospheric pressure and at 850-950°C. The ammonium ion destruction step is preferably carried out at a temperature of 70-90°C. The preparation of nitric acid by absorption of nitrogen dioxide in an aqueous solution will preferably be carried out at a temperature below 25°C. For an extensive description of the process conditions, reference is made to GB-A-1287302 and GB-A-1287303. The process conditions in the oxime synthesis zone (zone B) are described in GB-A-1279527. The process conditions in the hydroxylammonium salt synthesis zone (zone A) are described in US-A-3514254.

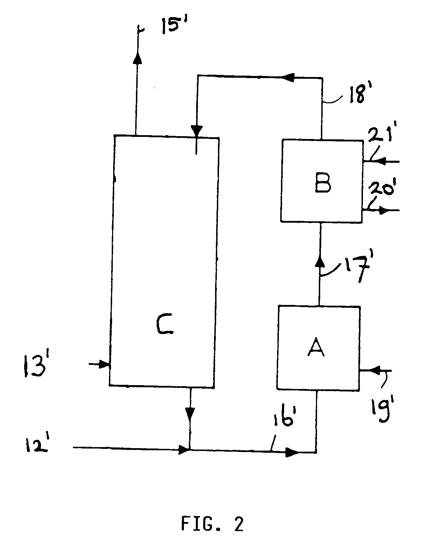

The process flows in Figure 2 are comparable with those in Figure 1 and are indicated with an apostrophe. Via line 13' a nitrogen oxide containing gas flow is supplied from a nearby nitric acid production facility. Nitric acid is supplied from the nitric acid production facility via line 12'. The hydroxylammonium salt synthesis zone A' and the oxime synthesis zone B' are the same as described above. Line 18', ammonium ion destruction zone C, which will in general consist of one reactor, and lines 16' and 17' complete the recycling system, wherein the reaction medium is recycled between zone A' and zone B'.

In an ammonium ion destruction zone the reaction medium, supplied via line 18', is contacted with the nitrogen oxide containing gas flow which is supplied via line 13', at a temperature which is preferably between 70 and 90°C. Via line 15' the residual gas flow, which still contains nitrogen oxides, is fed back to the nitric acid production process. Make-up nitric acid is supplied to the recycle mixture.

The invention will be further elucidated by means of the following example, without being restricted thereto.

### Example I

This example is carried out in accordance with a process as represented in Figure 2.

Via line 13, 874 kg/h nitrogen oxides (on dry basis) is drawn from a nitric acid plant and used for the destruction of ammonium ions in zone C. The nitrogen dioxide / nitrogen monoxide ratio is 4 : 1. Via line 12', 9400 kg/h HNO₃ (on dry basis) is drawn from the nitric acid plant and added to the reaction medium in line 16'. The residual production of the nitric acid production process amounts to 43,000 kg/h HNO₃ (on dry basis).

The efficiency of the ammonia combustion of the nitric acid production process amounts to 96% (mol NH₃ converted into nitrogen oxides).

## Claims

1. Process for the preparation and processing of a hydroxylammonium salt solution, wherein an aqueous acid reaction medium is kept in circulation between a hydroxylammonium salt synthesis zone and an oxime synthesis zone and wherein nitrate ions or nitrogen oxides to be converted into nitrate are continuously supplied, by way of nitrogen source for the formation of the hydroxylammonium salt, to the liquid being kept in circulation, which nitrate ions are catalytically reduced with hydrogen to hydroxylamine, and wherein ammonium ions, formed as a by-product in the reduction of the nitrate ions, are removed by conversion with nitrogen oxides, characterized in that the circulating liquid containing ammonia ions is contacted with a gas flow containing nitrogen oxides generated in catalytic combustion of ammonia, 0.01 to 5 wt.% of the nitrogen oxides formed in the combustion being used to convert ammonium ions into nitrogen, and in that the rest of the nitrogen oxide containing gas flow is used for the preparation of nitric acid.

2. Process according to claim 1, characterized in that the non-converted nitrogen oxides of the nitrogen oxide containing gas flow which is used for the destruction of ammonium ions are recycled into the nitric acid production process, and that the nitrate ions needed for the formation of the hydroxyl ammonium salt are also obtained from this nitric acid production process in the form of nitric acid.

3. Process according to claim 1, characterized in that the nitric acid production process has a capacity of 200 to 750 kt HNO₃/year (on dry basis).

4. Process according to any one of claims 1-3, characterized in that 0.5-3 wt.% of the nitrogen oxides is used to convert ammonium ions into nitrogen.

5. Process according to any one of claims 1-4, characterized in that the oxime is a cycloalkanone oxime with 6-12 carbon atoms.

6. Process according to claim 5, characterized in that the oxime is cyclohexanone oxime.

7. Process as substantially contained in the specification, drawings and the examples.
